(19)

Europäisches Patentamt

European Patent Office

Office européen des brevets

(11) **EP 4 225 477 B1**

(12) **EUROPEAN PATENT SPECIFICATION**

(45) Date of publication and mention of the grant of the patent:
**19.11.2025 Bulletin 2025/47**

(21) Application number: **21789633.1**

(22) Date of filing: **04.10.2021**

(51) International Patent Classification (IPC):
**B01D 67/00** (2006.01)    **A61M 1/36** (2006.01)
**B01D 69/02** (2006.01)    **B01D 69/08** (2006.01)
**B01D 69/14** (2006.01)    **B01D 71/08** (2006.01)
**B01D 71/68** (2006.01)

(52) Cooperative Patent Classification (CPC):
**B01D 67/00111; A61L 33/0035; A61M 1/3673;**
**B01D 67/0088; B01D 69/02; B01D 69/08;**
**B01D 69/144; B01D 71/08; B01D 71/68;**
B01D 2323/21839; B01D 2323/2187;
B01D 2323/36; B01D 2325/14; B01D 2325/16;
B01D 2325/36

(86) International application number:
**PCT/EP2021/077211**

(87) International publication number:
**WO 2022/078785 (21.04.2022 Gazette 2022/16)**

(54) **MEMBRANE WITH IMMOBILIZED ANTICOAGULANT AND PROCESS FOR PRODUCING SAME**

MEMBRAN MIT IMMOBILISIERTEM ANTIKOAGULANS UND VERFAHREN ZU IHRER HERSTELLUNG

MEMBRANE AYANT ANTICOAGULANT IMMOBILISÉ ET SON PROCÉDÉ DE PRODUCTION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.10.2020 EP 20201361**

(43) Date of publication of application:
**16.08.2023 Bulletin 2023/33**

(73) Proprietor: **Gambro Lundia AB**
**220 10 Lund (SE)**

(72) Inventors:
• **WESSLING, Matthias**
**52074 Aachen (DE)**
• **ALDERS, Michael**
**52066 Aachen (DE)**
• **ROSE, Ilka**
**52074 Aachen (DE)**
• **KATHER, Michael**
**52070 Aachen (DE)**

• **MENDA, Ralf**
**89250 Senden (DE)**
• **KRAUSE, Bernd**
**72414 Randendingen (DE)**
• **REMPFER, Martin**
**72810 Gomaringen (DE)**
• **KURBEL, Vivian Stefan**
**78667 Villingendorf (DE)**
• **STORR, Markus**
**70794 Filderstadt (DE)**

(74) Representative: **Perchenek, Nils**
**Gambro Dialysatoren GmbH**
**Legal and Intellectual Property**
**Holger Crafoord-Strasse 26**
**72379 Hechingen (DE)**

(56) References cited:
**WO-A1-2007/069983    WO-A1-2020/144244**
**US-A- 5 840 190**

- LINHARDT ROBERT ET AL: "Immobilization of Heparin: Approaches and Applications", CURRENT TOPICS IN MEDICINAL CHEMISTRY, vol. 8, no. 2, 1 January 2008 (2008-01-01), NL, pages 80 - 100, XP055781317, ISSN: 1568-0266, DOI: 10.2174/156802608783378891
- YANG M-C ET AL: "PROTEIN ADSORPTION AND PLATELET ADHESION OF POLYSULFONE MEMBRANE IMMOBILIZED WITH CHITOSAN AND HEPARIN CONJUGATE", POLYMERS FOR ADVANCED TECHNOLOGIES, WILEY & SONS, BOGNOR REGIS, GB, vol. 14, no. 2, 1 February 2003 (2003-02-01), pages 103 - 113, XP001163870, ISSN: 1042-7147, DOI: 10.1002/PAT.337
- XUE JIMIN ET AL: "Blood compatibility of polyethersulfone membrane by blending a sulfated derivative of chitosan", CARBOHYDRATE POLYMERS, APPLIED SCIENCE PUBLISHERS , LTD BARKING, GB, vol. 95, no. 1, 26 February 2013 (2013-02-26), pages 64 - 71, XP028585342, ISSN: 0144-8617, DOI: 10.1016/J.CARBPOL.2013.02.033

## Description

### Technical Field

The present disclosure relates to an anticoagulant-coated microporous hollow fiber membrane showing reduced thrombogenicity. The disclosure further relates to a method for producing the membrane and a filtration and/or diffusion device comprising the membrane.

### Background of the Invention

In extracorporeal blood circuits, systemic anticoagulation is generally used to prevent the coagulation of blood, i.e., the formation of microthrombi and blood clotting. Heparin is the most commonly used anticoagulant. Systemic anticoagulation with heparin is associated with increased bleeding risk and heparin is quite expensive. Therefore, membranes having the ability to immobilize heparin are highly desirable, as they reduce or even eliminate the need for systemic doses of heparin.

US 2003/0021826 A1 proposes binding an anticoagulation agent in a stable manner to the surface of semi-permeable support membranes essentially comprised of a copolymer of acrylonitrile and at least one anionic or anionizable monomer. The anticoagulation agent can exert its anticoagulating activity without being leached out into the blood or plasma during treatment by extracorporeal circulation and to reduce the quantity of anticoagulation agent used systemically in the patient during an extracorporeal blood treatment session. The surface of the semipermeable support membrane intended to be brought into contact with the blood or plasma is coated in succession with a cationic polymer carrying cationic groups which can form an ionic bond with anionic or anionizable groups of polyacrylonitrile, for example, polyethyleneimine (PEI), and an anticoagulation agent carrying anionic groups capable of forming an ionic bond with cationic groups of said cationic polymer (for example, heparin).

US 5 840 190 A discloses a surface modified biocompatible membrane. The membrane is comprised of polysulfone, polyvinylpyrrolidone and polyethyleneimine (PEI). The surface of the membrane is modified by reacting it with a bioactive compound and a coupling agent. In the working examples, heparin partly degraded by nitrous acid is covalently coupled to the membrane surface using sodium cyanoborohydride as coupling agent.

EP 1 024 886 A1 discloses a manufacturing method of a composite membrane having a hydrophilic coating layer on a hydrophobic support membrane wherein a hydrophobic support membrane is impregnated with an aqueous solution of a reacting agent to disperse the reacting agent on the surface and in the pores of the hydrophobic support membrane; and a hydrophilic polymer is thinly coated on the hydrophobic support membrane via reaction between the hydrophilic polymer and the reacting agent. The hydrophobic support membrane consists of polysulfone or polyetherimide; the hydrophilic polymer consists of sodium alginate, chitosan or polyvinyl alcohol. The reacting agent is sulfuric acid, when chitosan is used as a hydrophilic polymer; the concentration of the reacting agent in aqueous solution is in the range of 0.5-20%.

US 10 500 549 B2 discloses a multi-layer composite dialysis membrane comprising a base membrane based on polysulfone and at least one pore-forming hydrophilic additive which is a polyvinylpyrrolidone, short-chain glycol, triethylene glycol, propylene glycol, or polyethylene glycol/polyethylene oxide; and a functional layer arranged on the base membrane and formed from a layer of a polycationic bonding agent which is a polyethyleneimine, chitosan, polylysine, polyarginine, or polyornithine; and another layer of a polymeric polyanion which is a carboxylated polysaccharide or a sulfated polysaccharide selected from a dextran sulfate having a molecular mass (Mw) of 15 kDa to 1 MDa, a sulfated chitosan having a molecular mass (Mw) of 30 kDa to 750 kDa, a cellulose sulfate having a molecular mass (Mw) of 20 kDa to 1 MDa, and mixtures thereof.

US 2019/076787 A1 discloses a method for forming a microporous hollow fiber membrane involving extruding a polymer dope solution through an outer ring duct of a spinneret and simultaneously passing a precipitation fluid through an internal hollow core of the spinneret. The polymer dope solution comprises a membrane-forming polymer selected from polysulfone, polyarylsulfone, polyarylethersulfone, polyvinylidene fluoride, polyacrylonitrile or copolymers thereof, and optionally a hydrophilic polymer, such as polyvinylpyrrolidone or polyethylene glycol. The precipitation fluid comprises an additive with limited water solubility, such as polyurethane, chitosan or other N-containing polymers having an oxygen containing moiety. If chitosan is used as an additive, it can be protonated before introduction into the precipitation fluid using a weak acid, such as acetic acid, acetic acid anhydride, lactic acid, formic acid or a combination therof.

WO 2020/144244 A1 discloses an anticoagulant-coated microporous hollow fiber membrane showing reduced thrombogenicity. The hollow fiber membrane comprises a blend of i) polysulfone, polyethersulfone or polyarylethersulfone; ii) polyvinylpyrrolidone; and iii) at least one polymer bearing ammonium groups selected from the group consisting of polyvinylpyridines bearing ammonium groups, and copolymers of vinylpyridine and styrene bearing ammonium groups. An anticoagulant is grafted onto at least one surface of the membrane.

WO 2007/069983 A1 relates to a method for extracorporeal removal of a pathogenic microbe, an inflammatory cell or an inflammatory protein from mammalian blood. The method uses a device comprising heparin immobilized on a solid substrate. Preparatory example 4 discloses covalent attachment of heparin onto aminated polymeric surfaces. The polymeric surface is etched with an oxidizing agent and subsequently treated with a polyamine, polyethylene imine (PEI), or chitosan and further stabilized by ionic cross linking with heparin.

Linhardt, Robert et al.: "Immobilization of Heparin: Approaches and Applications", Current topics in medicinal chemistry, vol. 8, no.2, 2008-01-01, pp.80-100 examines and compares various techniques that have been used for the immobilization of heparin as well as applications of these immobilized heparins. Use of chitosan as a linker between reactive groups present on a substrate surface and heparin molecules are disclosed. The surface of a polysulfone membrane first was treated with ozone and then with aqueous acrylic acid (AA) solution containing $FeSO_4$ to produce carboxyl groups on the surface, chitosan molecules were coupled to the carboxyl groups using 1-Ethyl-3-(3-dimethylaminopropyl) carbodiimide (EDC), and subsequently heparin was coupled to the chitosan molecules using glutaraldehyde (GA).

It now has been found that anticoagulants like heparin can be immobilized on membranes prepared from a spinning solution comprising polyethersulfone, polyvinylpyrrolidone, and chitosan. The resulting heparin-coated microporous hollow fiber membranes show reduced thrombogenicity. Filtration and/or diffusion devices comprising the heparin-coated membranes reduce or even eliminate the need for systemic anticoagulation in extracorporeal blood circuits.

## Summary of the Invention

The present disclosure provides a porous hollow fiber membrane having an anticoagulant immobilized thereon. The membrane comprises i) a polysulfone, polyethersulfone, or polyarylethersulfone; ii) a polyvinylpyrrolidone; and iii) a chitosan. The present disclosure also provides a process for producing the porous hollow fiber membrane having an anticoagulant immobilized thereon. The present disclosure further provides filtration and/or diffusion devices comprising the porous hollow fiber membrane having an anticoagulant immobilized thereon. The filtration and/or diffusion devices, e.g., hemodialyzers, can be used in the extracorporeal treatment of blood, e.g., in hemodialysis, hemodiafiltration, and hemofiltration.

## Detailed Description of the Invention

In one aspect of the present invention, a porous hollow fiber membrane having an anticoagulant immobilized thereon is provided. The membrane comprises a blend of i) a polysulfone (PSU), polyethersulfone (PESU), or polyarylethersulfone (PAES); ii) a polyvinylpyrrolidone (PVP), and iii) a chitosan.

Examples of suitable polysulfones include polysulfones having a weight average molecular weight $M_w$ (determined by GPC) of about 40,000 to 80,000 Da. In one embodiment, a polysulfone having a weight average molecular weight $M_w$ in the range of from 45 to 65 kDa is used. An example is a polysulfone having a weight average molecular weight $M_w$ of 52 kDa (determined by GPC) and a polydispersity $M_w/M_n$ of 3.5. Another example is a polysulfone having a weight average molecular weight $M_w$ of 60 kDa (determined by GPC) and a polydispersity $M_w/M_n$ of 3.7.

Examples of suitable polyethersulfones include polyethersulfones having a weight average molecular weight $M_w$ (determined by GPC) of about 40,000 to 100,000 Da. In one embodiment, a polyethersulfone having a weight average molecular weight $M_w$ in the range of from 50 to 60 kDa is used. An example is a polyethersulfone having a weight average molecular weight $M_w$ of 58 kDa (determined by GPC) and a polydispersity $M_w/M_n$ of 3.3. In another embodiment, a polyethersulfone having a weight average molecular weight $M_w$ in the range of from 65 to 80 kDa is used. An example is a polyethersulfone having a weight average molecular weight $M_w$ of 75 kDa (determined by GPC) and a polydispersity $M_w/M_n$ of 3.4. In yet another embodiment, a polyethersulfone having a weight average molecular weight $M_w$ in the range of from 80 to 100 kDa is used. An example is a polyethersulfone having a weight average molecular weight $M_w$ of 92 kDa (determined by GPC) and a polydispersity $M_w/M_n$ of 3.0.

Suitable polyvinylpyrrolidones include homopolymers of vinylpyrrolidone having a weight average molecular weight $M_w$ in the range of from 50 kDa to 2,000 kDa. These homopolymers generally have a number average molecular weight $M_n$ in the range of from 14 kDa to 375 kDa. Examples of suitable polyvinylpyrrolidones for preparing the membranes of the invention are Luvitec® K30, Luvitec® K85, Luvitec® K90, and Luvitec® K90HM, respectively, all available from BASF SE.

One embodiment of the porous hollow fiber membrane of the present disclosure comprises a polyvinylpyrrolidone homopolymer having a weight average molecular weight $M_w$ of about 1,100 kDa; and a number average molecular weight $M_n$ of about 250 kDa.

A further embodiment of the porous hollow fiber membrane of the present disclosure comprises a polyvinylpyrrolidone homopolymer having a weight average molecular weight $M_w$ of about 1,400 kDa; and a number average molecular weight $M_n$ of about 325 kDa.

Chitosan is a linear polysaccharide composed of randomly distributed β-(1→4)-linked D-glucosamine (deacetylated unit) and N-acetyl-D-glucosamine (acetylated unit). Chitosan is produced commercially by deacetylation of chitin, which is the structural element in the exoskeleton of crustaceans (such as crab and shrimp) and cell walls of fungi. The degree of deacetylation (%DD) can be determined by NMR spectroscopy, and the %DD in commercial chitosan ranges from 60 to 100%. The weight average molecular weight $M_w$ of commercially produced chitosan generally is in the range between 3 kDa and 400 kDa.

One embodiment of the porous hollow fiber membrane of the present disclosure comprises a chitosan having a degree of deacetylation of at least 75%, i.e., at most 25% of the hydroxyl groups of the chitosan are acetylated. Another embodiment comprises a chitosan having a degree of deacetylation of at least 80%, e.g., at least 90%, or even at least 95%.

One embodiment of the porous hollow fiber membrane of the present disclosure comprises a chitosan having a weight average molecular weight $M_w$, determined by GPC analysis, in the range of from 5 kDa to 375 kDa, e.g., from 50 to 190 kDa.

In one embodiment, the porous hollow fiber membrane of the present disclosure comprises from 0.1 to 5 wt.%, relative to the total weight of the porous hollow fiber membrane, of chitosan. In a further embodiment, the porous hollow fiber membrane of the present disclosure comprises 0.5 to 3 wt.%, relative to the total weight of the porous hollow fiber membrane, of chitosan.

The hollow fiber membranes of the present disclosure either has a symmetric wall structure or an asymmetric wall structure. In one embodiment, the membrane wall has a symmetric sponge structure. In another embodiment, the membrane wall has an asymmetric sponge structure, i.e., the size of the pores in the hollow fiber wall increases from the inner surface of the membrane towards the outer surface. In yet another embodiment of the process, the membrane wall has an asymmetric wall structure and comprises a layer having a finger structure, i.e., featuring macrovoids having a volume-equivalent diameter of more than 5 $\mu$m.

In one embodiment, the hollow fiber membrane of the present disclosure has an inner diameter of from 150 to 250 $\mu$m, for instance, from 180 to 250 $\mu$m. In another embodiment, the inner diameter is in the range of from 185 $\mu$m to 195 $\mu$m. In yet another embodiment, the inner diameter is in the range of from 195 $\mu$m to 205 $\mu$m. In still another embodiment, the inner diameter is in the range of from 210 $\mu$m to 220 $\mu$m.

In one embodiment, the wall thickness of the hollow fiber membranes is in the range of from 15 $\mu$m to 60 $\mu$m. In one embodiment, the wall thickness is 33 $\mu$m to 37 $\mu$m. In another embodiment, the wall thickness is 38 to 42 $\mu$m. In still another embodiment, the wall thickness is 43 $\mu$m to 47 $\mu$m. In yet another embodiment, the wall thickness is 48 $\mu$m to 52 $\mu$m.

In one embodiment, the membrane has sieving coefficients, measured according to EN 1283 at 37°C in bovine plasma having a protein content of 60 g/l, for vitamin $B_{12}$ of 1.0, for inulin of 1.0, for $\beta_2$-microglobulin of at least 0.7, and for albumin of less than 0.01.

In one embodiment, the membrane has a molecular weight cutoff (MWCO) in whole blood in the range of from 10 kDa to 40 kDa. In a further embodiment, the average pore size in the selective layer of the membrane is in the range of from 2 to 5 nm.

In one embodiment, the membrane shows a hydraulic permeability (Lp) for water which is in the range of from $10 \cdot 10^{-4}$ cm$^3$/ (cm$^2$ $\cdot$bar $\cdot$sec) to 280 $\cdot 10^{-4}$cm$^3$/ (cm$^2$ $\cdot$bar $\cdot$sec) , for example from 15 $\cdot 10^{-4}$cm$^3$/ (cm$^2$ $\cdot$bar $\cdot$sec) to 130 $\cdot 10^{-4}$cm$^3$/ (cm$^2$ $\cdot$bar $\cdot$sec), or from 20 $\cdot 10^{-4}$cm$^3$/ (cm$^2$ $\cdot$bar $\cdot$sec) to 80 $\cdot 10^{-4}$cm$^3$/ (cm$^2 \cdot$bar$\cdot$sec).

The hollow fiber membrane of the present disclosure has an anticoagulant grafted onto its surface. In one embodiment, the anticoagulant is grafted onto the lumen surface of the hollow fiber membrane. In another embodiment, the anticoagulant is grafted onto the outer surface of the hollow fiber membrane. In still another embodiment, the anticoagulant is grafted onto the lumen surface, the outer surface, and the surface of the pore channels of the hollow fiber membrane. The anticoagulant forms an ionic bond with the ammonium groups of the protonated chitosan. The anticoagulant may comprise at least one compound of the glycosaminoglycan family with anticoagulation activity, and is preferably selected from the group consisting of unfractionated heparin, fractionated heparin, danaparoids, heparin derivatives, and mixtures of said products. In one embodiment, unfractionated heparin is used. The surface concentration of the deposited anticoagulant usually is in the range of from 1,000 to 30,000 IU/m$^2$, e.g., in the range of from 1,000 to 10,000 IU/m$^2$, or in the range of from 1,000 to 5,000 IU/m$^2$.

The present disclosure also provides a filtration and/or diffusion device comprising a plurality of porous hollow fiber membranes of the present disclosure. The filtration and/or diffusion device is a device for the extracorporeal purification of blood, e.g., a hemodialyzer. The surface area of a hemodialyzer comprising the hollow fiber membranes of the present disclosure may vary, but will usually be in a range of from 1.0 to 2.3 m$^2$. Hemodialyzers comprising the membrane of the present disclosure can be assembled as known in the art.

Sterilization of the devices will normally be effected by steam sterilization, irradiation with gamma rays, or using ETO. In one embodiment, the device is sterilized with steam at 121°C for at least 20 minutes. In another embodiment, gamma radiation dose used is in the range of from 25 to 50 kGy, for instance, 25 kGy. In still another embodiment, the device is sterilized with ETO.

The present disclosure also provides the use of the porous hollow fiber membranes of the present disclosure in hemodialysis, hemodiafiltration, or hemofiltration.

The present disclosure also provides a process for making the hollow fiber membrane having an anticoagulant grafted onto its surface. The process involves the production of a microporous hollow fiber support membrane and subsequent grafting of an anticoagulant onto at least one surface of the support membrane.

In one embodiment, the porous hollow fiber support membrane is prepared by a continuous solvent phase inversion spinning process comprising the steps of

a) forming a polymer solution comprising at least one polysulfone, polyethersulfone or polyarylethersulfone; at least one polyvinylpyrrolidone; at least one chitosan; and N-methyl-2-pyrrolidone;

b) extruding the polymer solution through an outer ring slit of a nozzle with two concentric openings into a precipitation bath; simultaneously

c) extruding a center fluid through the inner opening of the nozzle;

d) washing the membrane obtained; and subsequently

e) drying the membrane;

wherein the polymer solution comprises from 10 to 15 wt.%, relative to the total weight of the polymer solution, of polysulfone, polyethersulfone or polyarylethersulfone; and from 1 to 10 wt.%, relative to the total weight of the polymer solution, of polyvinylpyrrolidone; and from 0.05 to 0.6 wt.%, relative to the total weight of the solution, of chitosan.

The concentration of polyethersulfone in the polymer solution generally is in the range of from 10 to 15 wt.%, for instance, from 12 to 14 wt.%.

In one embodiment of the process, the polymer solution comprises a polyethersulfone having a weight average molecular weight $M_w$ in the range of from 90 to 95 kDa. An example is a polyethersulfone having a weight average molecular weight $M_w$ of 92 kDa and a polydispersity $M_w/M_n$ of 3. In another embodiment, the polymer solution comprises a polyethersulfone having a weight average molecular weight $M_w$ in the range of from 70 to 80 kDa. An example is a polyethersulfone having a weight average molecular weight $M_w$ of 75 kDa and a polydispersity $M_w/M_n$ of 3.4.

The concentration of polyvinylpyrrolidone in the polymer solution generally is in the range of from 1 to 10 wt.%, e.g., from 2 to 8 wt.%.

In one embodiment of the process, the polymer solution comprises 1 to 5 wt.% of a polyvinylpyrrolidone having a weight average molecular weight $M_w$ of 1,100 kDa.

In a further embodiment of the process, the polymer solution comprises 3 to 6 wt.% of a polyvinylpyrrolidone having a weight average molecular weight $M_w$ of 50 kDa.

In one embodiment of the process, the polymer solution comprises from 0.05 to 0.6 wt.%, e.g., 0.1 to 0.5 wt.%, or 0.2 to 0.4 wt.%, relative to the total weight of the solution, of chitosan.

In one embodiment, the chitosan has a degree of deacetylation of from 75% to 100%, and a weight average molecular weight, as determined by GPC, in the range of from 5 kDa to 375 kDa.

In a particular embodiment, the chitosan has a degree of deacetylation of from 85% to 95%, for instance, 90%, and a weight average molecular weight, as determined by GPC analysis, in the range of from 50 kDa to 190 kDa.

In one embodiment of the process, preparation of the polymer solution involves preparing an acidic aqueous solution of chitosan by dissolving chitosan in an aqueous solution of a weak organic acid. In one embodiment, the organic acid is lactic acid. In a further embodiment, a 90% w/w aqueous solution of lactic acid is used to dissolve the chitosan. In another embodiment, the organic acid is citric acid. In still another embodiment, the organic acid is acetic acid. In still another embodiment, the organic acid is tartaric acid.

In another embodiment of the process, preparation of the polymer solution involves preparing an acidic solution of chitosan by dissolving chitosan in pure lactic acid.

It has been found that lactic acid has advantages over other weak organic acids like acetic acid, citric acid, or tartaric acid. As lactic acid is liquid at room temperature, no water needs to be added to prepare the chitosan solution. It has been found that polymer solutions produced using citric acid show poor long-term stability and fibers produced using the polymer solution tend to rupture during the spinning process. Chitosan solutions in aqueous acetic acid form gels on addition of polyvinylpyrrolidone; and polyvinylpyrrolidone is insoluble in chitosan solutions in aqueous tartaric acid.

In one embodiment, the concentration of chitosan in the lactic acid solution is in the range of from 0.2 wt.% to 5 wt.%, for instance, from 1 wt.% to 4 wt.%, or from 1.5 wt.% to 3 wt.%.

The chitosan solution is mixed with N-methyl-2-pyrrolidone, and polyvinylpyrrolidone is added. After the polyvinylpyrrolidone has dissolved, polyethersulfone is added and the mixture is stirred until a clear solution has formed.

In one embodiment, the polymer solution comprises from 1 to 4 wt.%, e.g., 1.5 to 3 wt.%, relative to the total weight of the solution, of water. In another embodiment, the polymer solution does not comprise added water.

In one embodiment of the process for preparing the membrane, the center fluid comprises 40 to 60 wt.% of water and 40 to 60 wt.% of NMP, for instance, 50 to 60 wt.% of water and 40 to 50 wt.% of NMP, or 52 to 55 wt.% of water and 45 to 48 wt.% of NMP, e.g., 54 wt.% of water and 46 wt.% of NMP, relative to the total weight of the center fluid.

In one embodiment of the process, the precipitation bath is comprised of water. In one embodiment of the process, the precipitation bath has a temperature in the range of from 10 to 30°C, for instance, 15 to 25°.

In one embodiment of the process for preparing the membrane, the temperature of the spinneret is in the range of from 40 to 60°C, e.g., from 52 to 56°C.

In one embodiment of the process, the distance between the opening of the nozzle and the precipitation bath is in the range of from 10 to 120 cm, e.g., from 90 to 110 cm.

In one embodiment of the process, the spinning speed is in the range of from 20 to 80 m/min, e.g., from 30 to 50 m/min.

The membrane then is washed to remove residual solvent and low molecular weight components. In a particular embodiment of a continuous process for producing the membrane, the membrane is guided through several water baths. In certain embodiments of the process, the individual water baths have different temperatures. For instance, each water bath may have a higher temperature than the preceding water bath.

Subsequently, an anticoagulant is grafted onto at least one surface of the membrane. In one embodiment, the surface is the lumen surface of the membrane, i.e., its interior wall surface. In another embodiment, the surface is the outer surface of the membrane, i.e., its exterior wall surface. In still another embodiment, both the inner wall surface and the surface of the pore channels within the membrane are grafted with the anticoagulant. In yet another embodiment, both the outer wall surface and the surface of the pore channels within the membrane are grafted with the anticoagulant. In still another embodiment, the inner wall surface, the pore channels within the membrane, and the outer wall surface of the membrane are grafted with the anticoagulant.

The anticoagulant forms an ionic bond with the ammonium groups present on the surface of the membrane. The anticoagulant is attached to the membrane surface by electrostatic interaction between the negatively charged anticoagulant and the positively charged membrane surface. In one embodiment, the anticoagulant comprises at least one member of the gly-coaminoglycane family having anticoagulation activity. In a further embodiment, the anticoagulant is selected from the group consisting of unfractionated heparin, fractionated heparin, danaparoids, heparin derivatives, and mixtures of said products. In one embodiment, the anticoagulant is unfractionated heparin. In one embodiment, the concentration of the anticoagulation agent on the membrane surface after the grafting step is in the range of from 1,000 to 30,000 IU/m$^2$, e.g., in the range of from 1,000 to 10,000 IU/m$^2$, or in the range of from 1,000 to 5,000 IU/m$^2$, for instance, from 1,500 to 3,500 IU/m$^2$.

The grafting can be performed by contacting an aqueous solution of the anticoagulant with the support membrane surface. In one embodiment, the support membrane is flushed with an aqueous solution of the anticoagulant and subsequently rinsed with water or saline to remove excess anticoagulant. In another embodiment, the support membrane is flushed with an aqueous solution of the anticoagulant and subsequently dried to evaporate the solvent. In still another embodiment, the support membrane is soaked with an aqueous solution of the anticoagulant, making use of the capillary force generated by the hollow fiber.

The grafting can be performed before or after the membrane has been incorporated into a diffusion and/or filtration device. In other words, the grafting can also be performed on a diffusion and/or filtration device comprising the porous hollow fiber membrane bearing positive charges of the present disclosure. For instance, a diffusion and/or filtration device, e.g., a hemodialyzer comprising a bundle of porous hollow fiber membrane bearing positive charges can be flushed with an aqueous solution of the anticoagulant to effect the grafting; and subsequently the device is rinsed with water or saline to remove excess anticoagulant. In another embodiment, the device is flushed with an aqueous solution of the anticoagulant to effect the grafting; and subsequently the membranes in the device are dried and the solvent is evaporated.

After the grafting, the membrane can be post-processed. Examples of post-processing include extensive rinsing of the membrane to remove loosely bound anticoagulant; radiation treatment of the membrane to cross-link the anticoagulant with other polymers present in the membrane; drying of the membrane; cross-linking polymer chains of polymers present in the membrane by heating the membrane.

The present disclosure thus provides a simple process for producing membranes coated with anticoagulants like heparin. An advantage of the membrane of the present disclosure is that no intermediate or priming layer between the base material and the anticoagulant coating is present, and the process for making the membrane does not require the additional steps for forming such a priming layer. The process of the present disclosure also does not involve an additional chemical reaction step for covalently coupling the anticoagulant to the membrane surface and consequently does not involve the use of further coupling agents. The present disclosure provides a simple, scalable procedure for low-cost manufacturing of heparin-coated membranes.

The subject matter of the present disclosure is further described in the following working examples.

**Methods**

**Preparation of mini-modules**

Mini-modules [= fibers in a housing] are prepared by cutting the fibers to a length of 20 cm, drying the fibers for 1 h at 40°C and < 100 mbar and subsequently transferring the fibers into the housing. The ends of the fibers are potted with polyurethane. After the polyurethane has hardened, the ends of the potted membrane bundle are cut to reopen the fibers. The mini-module ensures protection of the fibers. In the present examples, mini modules having an effective membrane (lumen) surface A of 360 cm$^2$ were used.

**Hydraulic Permeability (Lp) of mini-modules**

The hydraulic permeability of a mini-module is determined by pressing a defined volume of water under pressure through the mini-module, which has been sealed on one side, and measuring the required time. The hydraulic permeability is calculated from the determined time t, the effective membrane surface area A, the applied pressure p and the volume of water pressed through the membrane V, according to equation (1):

$$Lp = V / [p \cdot A \cdot t] \qquad (1)$$

The effective membrane surface area A is calculated from the fiber length and the inner diameter of the fiber according to equation (2)

$$A = \pi \cdot d_i \cdot l \cdot [cm^2] \qquad (2)$$

with

$d_i$ = inner diameter of fiber [cm]
l = effective fiber length [cm]

The mini-module is wetted thirty minutes before the Lp-test is performed. For this purpose, the mini-module is put in a box containing 500 mL of ultrapure water. After 30 minutes, the mini-module is transferred into the testing system. The testing system consists of a water bath that is maintained at 37°C and a device where the mini-module can be mounted. The filling height of the water bath has to ensure that the mini-module is located underneath the water surface in the designated device.

In order to avoid that a leakage of the membrane leads to a wrong test result, an integrity test of the mini-module and the test system is carried out in advance. The integrity test is performed by pressing air through the mini-module that is closed on one side. Air bubbles indicate a leakage of the mini-module or the test device. It has to be checked if the leakage is due to an incorrect mounting of the mini-module in the test device or if the membrane leaks. The mini-module has to be discarded if a leakage of the membrane is detected. The pressure applied in the integrity test has to be at least the same value as the pressure applied during the determination of the hydraulic permeability in order to ensure that no leakage can occur during the measurement of the hydraulic permeability because the pressure applied is too high.

**Heparin coating of hollow fibers in mini-modules**

i) A solution of heparin (Heparin sodium 194 IU/mg, Celsus Laboratories Inc., Cincinnati, USA) in isotonic saline solution (0.9%) having a concentration of 200 IU/ml was prepared. The dialysate side of the minimodule was closed to ensure that no filtration from blood side to dialysate side could take place. 25 ml of the heparin solution were recirculated through the blood side of the minimodule for 30 min at a flow rate of 1 ml/min. After coating, the minimodules were subsequently rinsed with 300 ml of isotonic saline solution and 100 ml of water at a flow rate of 20 ml/min. The module was then dried by flushing the blood side of the minimodule with compressed air for 80 min.

ii) A solution of heparin (Heparin sodium 194 IU/mg, Celsus Laboratories Inc., Cincinnati, USA) in ultrapure water (Milli-Q® Advantage A10, Merck KGaA, Darmstadt, Germany) having a concentration of 200 IU/ml was prepared. The dialysate side of the minimodule was closed to ensure that no filtration from blood side to dialysate side could take place. 25 ml of the heparin solution were recirculated through the blood side of the minimodule for 120 min at a flow rate of 1 ml/min. After coating, the minimodules were subsequently rinsed with 300 ml of isotonic saline solution and 100 ml of water at a flow rate of 20 ml/min. The module was then dried by flushing the blood side of the minimodule with compressed air for 80 min.

## Quantitative determination of immobilized heparin

A glycine buffer was prepared from 3.84 g/l glycine (Merck KGaA, Darmstadt, Germany), 2.8 g/l NaCl (Merck KGaA, Darmstadt, Germany) and water. The pH was adjusted to 11 $\pm$ 0.1 with a 30% NaOH solution (VWR, Darmstadt, Germany). The minimodule was rinsed with the glycine buffer at a flow rate of 2.3 ml/min. After 10 minutes, the minimodule was emptied completely. From the extract after 10 minutes, a sample was collected and heparin concentration in the sample was determined using an Azure A assay.

Azure A is a blue colored, metachromatic dye which changes the color from blue to purple-red when binding to sulfated polysaccharides such as heparin. This color change is measured photometrically at a wavelength of 630 nm. The heparin concentration was determined based on a standard curve which covered a concentration range from 0 mg/l to 12 mg/l heparin. Control samples with known concentrations of 1.2 mg/l, 6 mg/l and 10.8 mg/l of heparin were measured simultaneously each time. 200 $\mu$l of the aqueous Azure A (Dye content ~80%, Sigma-Aldrich Chemie GmbH, Steinheim, Germany) solution with a concentration of 25 mg/l Azure A were added to 100 $\mu$l of the standard samples, control samples and samples to be measured, respectively, in microplate wells. After storing the microplate in the dark for 15 minutes, the measurement was performed using a microplate photometer (EL 808 Ultra Microplate Reader, BioTek Instruments Inc., Winooski, USA).

## Determination of heparin release

First, the minimodule was rinsed with about 150 ml of isotonic saline solution (0.9%) at 37°C. Then a reservoir of 30 ml of solvent/detergent treated pooled human plasma comprising 45-70 g/l human plasma proteins, 4.4-7.4 g/l sodium citrate dihydrate, 0.3-1.2 g/l sodium dihydrogen-phosphate dihydrate, and 4.0-6.0 g/l glycine, and showing a clotting factor activity of at least 0.5 IU/ml (Octaplas® LG, Octapharma Pharmazeutika Produktionsgesellschaft mbH, A-1100 Vienna, Austria) was recirculated through the minimodule at a flow rate of 9 ml/min for 120 minutes. Samples were taken at 0 min, 60 Min, and 120 min. Heparin concentration in the samples was determined using a Heparin Anti-Xa Chromogenic Assay (Stachrom® Heparin; Stago Deutschland GmbH, 40474 Düsseldorf, Germany).

## Determination of hemocompatibility

The hemocompatibility of the membranes was investigated by the perfusion of minimodules with PRP (platelet-rich human plasma comprising 100,000 thrombocytes per $\mu$l) *in vitro.*

First, the blood side of the minimodule was rinsed with about 250 ml of isotonic saline solution (0.9%) at 37°C; then the filtrate side of the minimodule was rinsed with about 120 ml of isotonic saline solution (0.9%) at 37°C and subsequently closed with stoppers. A reservoir of 50 ml of PRP comprising 0.05 IU/ml heparin was recirculated through the minimodule at a flow rate of 9 ml/min for 120 minutes. At 0 min, 10 min, 20 min, 40 min, 60 Min, 90 min, and 120 min, two samples of 450 $\mu$l volume were taken. In each case, one of the two samples was transferred into a micro tube (Micro Tube 1,5 ml EASY CAP, Sarstedt AG & Co. KG, Nümbrecht, Germany) containing 50 $\mu$l of a 10 wt.% trisodium citrate solution to inhibit further coagulation of the sample and stored in an ice bath. The other sample was transferred into a micro tube (Micro Tube 1,5 ml EASY CAP, Sarstedt AG & Co. KG, Nümbrecht, Germany) containing 50 $\mu$l of a CTAD (citrate-theophylline-adenosine-dipyridamole) solution to inhibit further coagulation of the sample and stored in an ice bath.

### Determination of platelet drop

Thrombocyte content of the plasma samples was analysed using a cell counting system (XP-300, Sysmex Deutschland GmbH, 22848 Norderstedt, Germany). Data is shown relative to the starting concentration of 100.000 THR/$\mu$L, which corresponds to 100%.

### Determination of PF-4

The formation of human platelet factor 4 (PF-4) was examined by means of a sandwich enzyme-linked immunosorbent assay (ELISA) (Asserachrom® PF4, Stago Deutschland GmbH, 40474 Düsseldorf, Germany).

After thawing, the aliquoted plasma samples comprising CTAD were diluted with human plasma. The dilution factor was 1:50. Standard samples (range from 2 to 60 $\mu$g/l), control samples and the plasma for dilution were assayed in duplicates; while for the samples single determination was sufficient. 50 µl of diluent and 50 µl of the samples were transferred into a well of a microplate and the PF-4 bonded to the antibodies on the microplate. After a washing process, 100 µl of the second enzyme-conjugated antibody were added and removed by washing after 30 minutes of incubation. A reaction with 3,3',5,5'-Tetramethylbenzidin (TMB) was measured photometrically (EL 808 Ultra Microplate Reader, BioTek Instruments Inc., Winooski/USA) at 450 nm.

Determination of human TAT complex

The activation of coagulation by the membrane surface was examined by the quantitative determination of thrombin-antithrombin III complex (TAT) in human plasma by means of a sandwich enzyme-linked immunosorbent assay (ELISA) (Enzygnost® TAT micro, Siemens Healthcare Diagnostics Products GmbH, Germany).

After thawing, the aliquoted plasma samples comprising trisodium citrate were diluted with human plasma. The dilution factor was chosen depending on the expected TAT concentration and varied between 1:1 and 1:40. Standard samples (range from 2 to 60 µg/l), control samples and the plasma for dilution were assayed in duplicates; while for the samples single determination was sufficient. 50 µl of diluent and 50 µl of the samples were transferred into a well of a microplate and the TAT complex bonded to the antibodies on the microplate. After a washing process, 100 µl of the second enzyme-conjugated antibody were added and removed by washing after 30 minutes of incubation. An enzymatic reaction which occurs by addition of chromogen was measured photometrically (EL 808 Ultra Microplate Reader, BioTek Instruments Inc., Winooski/USA) at 490 nm within one hour.

**Examples**

**Comparative Example 1**

Minimodules were prepared using fibers taken from a commercial dialyzer (Revaclear®, Gambro Lundia AB). The fibers have an inner diameter of 190 µm and a wall thickness of 35 µm and are comprised of a blend of polyarylethersulfone and polyvinylpyrrolidone.

Hemocompatibility of the membrane was investigated as described above. After 90 minutes, clotting was observed. Due to the clotting, the experiment was terminated after 110 minutes. Platelet drop, formation of PF-4 and human TAT complex were determined as described above. The results are summarized in Table 1.

Table 1

| t [min] | 0 | 10 | 20 | 40 | 60 | 90 | 110 |
|---|---|---|---|---|---|---|---|
| PLT [%] | 100 | 97 | 97 | 96 | 87 | 88 | 57 |
| PF-4 [IU/ml] | 1415 | 1406 | 1348 | 1615 | 2365 | 4041 | 3753 |
| TAT [µg/l] | 119 | 109 | 104 | 140 | 213 | 407 | 592 |

**Comparative Example 2**

Minimodules were prepared using fibers taken from a commercial dialyzer (Evodial®, Gambro Lundia AB) that had been rinsed with a mixture of 60 wt.% glycerol and 40 wt.% water 100 ml/min recirculating for 60 min and then dried with compressed air for 2 hrs. The fibers have an inner diameter of 210 µm and a wall thickness of 42 µm and are comprised of an acrylonitrile and sodium methallyl sulfonate copolymer grafted with polyethyleneimine and coated with 3,000 IU/m$^2$ unfractionated heparin.

Hemocompatibility of the membrane was investigated as described above. Platelet drop, formation of PF-4 and human TAT complex were determined as described above. The results are summarized in Table 2.

Table 2

| t [min] | 0 | 10 | 20 | 40 | 60 | 90 | 120 |
|---|---|---|---|---|---|---|---|
| PLT [%] | 100 | 101 | 103 | 108 | 107 | 109 | 103 |
| PF-4 [IU/ml] | 1092 | 988 | 958 | 1186 | 1251 | 1349 | 1576 |
| TAT [µg/l] | 24 | 23 | 22 | 17 | 20 | 24 | 25 |

**Preparation of a polymer solution**

For 4,500 g (= 100% w/w) of polymer solution, 11.25 g (0.25% w/w) of chitosan (Chitosan 90/100/A1, Kraeber & Co GmbH,25474 Ellerbek, Germany) were dissolved in 720 g (16% w/w) of a 90% w/w aqueous solution of lactic acid (GPR Rec-tapur®, VWR International GmbH, 64295 Darmstadt, Germany). The chitosan solution was mixed with 3,048.75g (67.75% w/w) N-methyl-2-pyrrolidone, and 90.00g (2% w/w) of a polyvinylpyrrolidone having a weight average molecular weight of about 1,100 kDa (Luvitec® K85, BASF SE) was added. After the polyvinylpyrrolidone had dissolved, 630.00 g (14% w/w) of polyethersulfone (Ultrason® E 6020, BASF SE) was added and the mixture was stirred until a clear solution had formed. It is also possible to dissolve PVP and polyethersulfone in NMP and then add the solution of chitosan in lactic acid in a second step.

**Example 1**

A solution of 14.0% w/w polyethersulfone having a weight average molecular weight of about 75 kDa (Ultrason® E 6020, BASF SE); 2.0% w/w PVP having a weight average molecular weight of about 1,100 kDa (Luvitec® K85, BASF SE); 0.25% w/w of chitosan (Chitosan 90/100/A1, Kraeber & Co GmbH, 25474 Ellerbek, Germany); 14.4% w/w lactic acid; 1.6% water and 67,75% w/w NMP was extruded through the outer ring slit of a spinneret with two concentric openings (diameter of outer boundary of ring slit: 500 $\mu$m; diameter of inner boundary of ring slit: 350 $\mu$m; diameter of center bore: 180 $\mu$m) into a coagulation bath containing water. A solution containing 50% w/w water and 50% w/w NMP was used as the center fluid and extruded through the inner orifice of the spinneret. The temperature of the spinneret was 48°C, the temperature in the spinning shaft was 45°C, the air gap was 100 cm, and the coagulation bath had room temperature. The fibers were spun at a speed of 40 m/min. The fibers subsequently were guided through a sequence of water baths containing demineralized water at temperatures in the range of from 50°C to 75°C.

The fiber obtained had an inner diameter of 190 $\mu$m and a wall thickness of 35 $\mu$m. The membrane showed an asymmetric sponge structure, with average pore size increasing in the direction from the inner wall surface towards the outer wall surface.

Mini-modules were prepared as described above and hydraulic permeability of the fibers was tested as described above. The Lp of the mini-modules was determined to be $(45\pm5) \cdot 10^{-4}$ cm$^3$/ (cm$^2$ ·bar ·sec) (n=2).

A number of mini-modules were sterilized with steam at 121°C for 20 min. After sterilization, Lp of the mini-modules was determined to be $(70\pm3) \cdot 10^{-4}$ cm$^3$/ (cm$^2$ ·bar ·sec) (n=2). The sieving coefficients of the sterilized membranes were $(31\pm1)$% for myoglobin and $(0.7\pm0.1)$% for albumin (n=2).

The membranes of three minimodules were coated with heparin according to procedure i) described above and the amount of heparin immobilized on the membranes was determined as described above. After 10 minutes of extraction, $(92\pm4)$ IU of heparin were extracted from each minimodule (n=3).

Heparin release from three minimodules (n=3) was tested as described above. Heparin concentration in plasma at 0 min was below the detection limit (<0.1 IU/ml). After 60 minutes of extraction, heparin concentration in the plasma sample was below the detection limit (<0.1 IU/ml) for the first and the second minimodule and 0.1 IU/ml for the third minimodule. After 120 minutes of extraction, heparin concentration in the plasma sample was below the detection limit (<0.1 IU/ml) for the first and the third minimodule and 0.3 IU/ml for the second minimodule.

The Lp of the heparin-coated mini-modules was determined to be $(65\pm2) \cdot 10^{-4}$ cm$^3$/ (cm$^2$ ·bar·sec) (n=3). The sieving coefficients of the heparin-coated membranes were $(33\pm1)$% for myoglobin and $(0.8\pm0.1)$ % for albumin (n=3).

**Example 2**

A solution of 14.0% w/w polyethersulfone having a weight average molecular weight of about 75 kDa (Ultrason® E 6020, BASF SE); 2.0% w/w PVP having a weight average molecular weight of about 1,100 kDa (Luvitec® K85, BASF SE); 0.25% w/w of chitosan (Chitosan 90/100/A1, Kraeber & Co GmbH, 25474 Ellerbek, Germany); 14.4% w/w lactic acid; 1.6% water and 67.75% w/w NMP was extruded through the outer ring slit of a spinneret with two concentric openings (diameter of outer boundary of ring slit: 700 $\mu$m; diameter of inner boundary of ring slit: 350 $\mu$m; diameter of center bore: 180 $\mu$m) into a coagulation bath containing water. A solution containing 50% w/w water and 50% w/w NMP was used as the center fluid and extruded through the inner orifice of the spinneret. The temperature of the spinneret was 49°C, the temperature in the spinning shaft was 45°C, the air gap was 100 cm, and the coagulation bath had room temperature. The fibers were spun at a speed of 30 m/min. The fibers subsequently were guided through a sequence of water baths containing demineralized water at temperatures in the range of from 50°C to 75°C.

The fiber obtained had an inner diameter of 190 $\mu$m and a wall thickness of 35 $\mu$m. The membrane showed an asymmetric sponge structure, with average pore size increasing in the direction from the inner wall surface towards the outer wall surface.

Mini-modules were prepared as described above and hydraulic permeability of the fibers was tested as described above. The Lp of the mini-modules was determined to be $(44\pm1)\cdot10^{-4}$ cm$^3$/ (cm$^2$ ·bar ·sec) (n=3). The sieving coefficients of the membranes were $(31\pm0.5)$% for myoglobin and $(1.5\pm0.0)$% for albumin (n=3).

A number of mini-modules were sterilized with steam at 121°C for 20 min. After sterilization, Lp of the mini-modules was determined to be $(82\pm4)\cdot10^{-4}$ cm$^3$/(cm$^2$·bar·sec) (n=3). The sieving coefficients of the sterilized membranes were $(39\pm2)$% for myoglobin and $(1.7\pm0.1)$% for albumin (n=3).

The membranes of a minimodule were coated with heparin according to procedure i) described above and hemocompatibility of the membrane was investigated as described above. Platelet drop, formation of PF-4 and human TAT complex were determined as described above. The results are summarized in Table 3.

Table 3

| t [min] | 0 | 10 | 20 | 40 | 60 | 90 | 120 |
|---|---|---|---|---|---|---|---|
| PLT [%] | 100 | 105 | 111 | 99 | 106 | 95 | 104 |
| PF-4 [IU/ml] | 1059 | 992 | 1088 | 1271 | 1326 | 1640 | 1808 |
| TAT [μg/l] | 29 | 26 | 25 | 20 | 27 | 30 | 30 |

## Example 3

A solution of 14.0% w/w polyethersulfone having a weight average molecular weight of about 75 kDa (Ultrason® E 6020, BASF SE); 2.0% w/w PVP having a weight average molecular weight of about 1,100 kDa (Luvitec® K85, BASF SE); 0.1% w/w of chitosan (Chitosan 90/100/A1, Kraeber & Co GmbH, 25474 Ellerbek, Germany); 14.4% w/w lactic acid; 1.6% water and 67,9% w/w NMP was extruded through the outer ring slit of a spinneret with two concentric openings (diameter of outer boundary of ring slit: 500 μm; diameter of inner boundary of ring slit: 350 μm; diameter of center bore: 180 μm) into a coagulation bath containing water. A solution containing 50% w/w water and 50% w/w NMP was used as the center fluid and extruded through the inner opening of the spinneret. The temperature of the spinneret was 49°C, the temperature in the spinning shaft was 45°C, the air gap was 100 cm, and the coagulation bath had room temperature. The fibers were spun at a speed of 40 m/min. The fibers subsequently were guided through a sequence of water baths containing demineralized water at temperatures in the range of from 50°C to 75°C.

The fiber obtained had an inner diameter of 190 μm and a wall thickness of 35 μm. The membrane showed an asymmetric sponge structure, with average pore size increasing in the direction from the inner wall surface towards the outer wall surface.

The Lp of the mini-modules was $(73\pm1)\cdot10^{-4}$ cm$^3$/ (cm$^2$ ·bar ·sec) (n=3). After steam sterilization, Lp of the mini-modules was $(91\pm1)\cdot10^{-4}$ cm$^3$/ (cm$^2$ ·bar ·sec) (n=2). The sieving coefficients of the sterilized membranes were (4411)% for myoglobin and $(0.8\pm0.0)$ % for albumin (n=2).

The membranes of three minimodules were coated with heparin according to procedure ii) described above and the amount of heparin immobilized on the membranes was determined as described above. After 10 minutes of extraction, $(43\pm2)$ IU of heparin were extracted from each minimodule (n=3).

The Lp of the heparin-coated mini-modules was determined to be $(82\pm2)\cdot10^{-4}$ cm$^3$/ (cm$^2$ ·bar·sec) (n=2). The sieving coefficients of the heparin-coated membranes were $(42\pm1)$% for myoglobin and $(0.8\pm0.0)$ % for albumin (n=2).

## Example 4

A solution of 14.0% w/w polyethersulfone having a weight average molecular weight of about 75 kDa (Ultrason® E 6020, BASF SE); 2.0% w/w PVP having a weight average molecular weight of about 1,100 kDa (Luvitec® K85, BASF SE); 0.4% w/w of chitosan (Chitosan 90/100/A1, Kraeber & Co GmbH, 25474 Ellerbek, Germany); 14.4% w/w lactic acid; 1.6% water and 67.6% w/w NMP was prepared. In order to reduce viscosity of the polymer solution, 30 wt. % NMP, relative to the total weight of the polymer solution, was added. The diluted polymer solution was extruded through the outer ring slit of a spinneret of a spinneret with two concentric openings (diameter of outer boundary of ring slit: 500 μm; diameter of inner boundary of ring slit: 350 μm; diameter of center bore: 180 μm) into a coagulation bath containing water. A solution containing 60% w/w water and 40% w/w NMP was used as the center fluid and extruded through the inner opening of the spinneret. The temperature of the spinneret was 45°C, the temperature in the spinning shaft was 42°C, the air gap was 100 cm, and the coagulation bath had room temperature. The fibers were spun at a speed of 40 m/min. The fibers subsequently were guided through a sequence of water baths containing demineralized water at temperatures in the range of from 50°C to 75°C.

The fiber obtained had an inner diameter of 190 μm and a wall thickness of 35 μm. The membrane showed a finger structure, i.e., macro-voids extending in the direction towards the outer wall surface.

The Lp of the mini-modules was $(91\pm6)\cdot10^{-4}$ cm$^3$/ (cm$^2$ ·bar·sec) (n=3). The sieving coefficients of the membranes were (21±2) % for myoglobin and (0.5±0.0)% for albumin (n=3).

The membranes of three minimodules were coated with heparin according to procedure ii) described above and the amount of heparin immobilized on the membranes was determined as described above. After 10 minutes of extraction, (18±1) IU of heparin were extracted from each minimodule (n=3).

**Claims**

1. A porous hollow fiber membrane having an anticoagulant immobilized thereon; the membrane comprising a blend of i) a polysulfone, polyethersulfone or polyarylethersulfone; ii) a polyvinylpyrrolidone; and iii) a chitosan.

2. The membrane of claim 1, wherein the anticoagulant is a glycosaminoglycan.

3. The membrane of claim 2, wherein the anticoagulant is unfractionated heparin.

4. The membrane of any one of claims 1 to 3, wherein the concentration of the anticoagulant on the porous hollow fiber is in the range of from 1,000 to 5,000 $IU/m^2$.

5. The membrane of any one of claims 1 to 4, comprising from 0.1 to 5 wt.%, relative to the total weight of the membrane, of chitosan.

6. The membrane of any one of claims 1 to 5, wherein the chitosan has a weight average molecular weight $M_w$, determined by GPC analysis, in the range of from 5 to 375 kDa.

7. The membrane of any one of claims 1 to 6, wherein the chitosan has a degree of deacetylation in the range of from 85 to 95%.

8. A process for preparing a porous hollow fiber membrane having an anticoagulant immobilized thereon, comprising the production of a microporous hollow fiber support membrane and subsequent grafting of an anticoagulant onto at least one surface of the support membrane, wherein the production of the microporous hollow fiber support membrane comprises the steps of

   a) forming a polymer solution comprising at least one polysulfone, polyethersulfone or polyarylethersulfone; at least one polyvinylpyrrolidone; at least one chitosan; and N-methyl-2-pyrrolidone;
   b) extruding the polymer solution through an outer ring slit of a nozzle with two concentric openings into a precipitation bath; simultaneously
   c) extruding a center fluid through the inner opening of the nozzle;
   d) washing the membrane obtained; subsequently
   e) drying the membrane;

   wherein the polymer solution comprises from 10 to 15 wt.%, relative to the total weight of the polymer solution, of polysulfone, polyethersulfone or polyarylethersulfone; and from 1 to 10 wt.%, relative to the total weight of the polymer solution, of polyvinylpyrrolidone; and from 0.05 to 0.6 wt.%, relative to the total weight of the solution, of chitosan.

9. The process of claim 8, wherein the chitosan has a degree of deacetylation of from 75% to 100%, and a weight average molecular weight $M_w$, determined by GPC analysis, in the range of from 5 kDa to 375 kDa.

10. The process of claim 8 or 9, wherein forming the polymer solution involves dissolving the chitosan in lactic acid.

11. The process of claim 10, wherein the chitosan is dissolved in a 90% w/w aqueous solution of lactic acid.

12. The process of any one of claims 8 to 11, wherein the anticoagulant is grafted onto the at least one surface of the support membrane by contacting an aqueous solution of the anticoagulant with the at least one surface of the support membrane.

13. The process of any one of claims 8 to 12, wherein the anticoagulant is unfractionated heparin.

14. A filtration and/or diffusion device comprising a plurality of porous hollow fiber membranes according to any one of claims 1 to 7.

15. Use of a porous hollow fiber membrane according to any one of claims 1 to 7 in hemodialysis, hemodiafiltration, or hemofiltration.

**Patentansprüche**

1. Poröse Hohlfasermembran, auf der ein Antikoagulans immobilisiert ist; die Membran umfassend eine Mischung aus i) einem Polysulfon, Polyethersulfon oder Polyarylethersulfon; ii) einem Polyvinylpyrrolidon; und iii) einem Chitosan.

2. Membran nach Anspruch 1, worin das Antikoagulans ein Glycosaminoglycan ist.

3. Membran nach Anspruch 2, worin das Antikoagulans unfraktioniertes Heparin ist.

4. Membran nach einem der Ansprüche 1 bis 3, worin die Konzentration des Antikoagulans auf der porösen Hohlfaser im Bereich von 1.000 bis 5.000 IU/m$^2$ beträgt.

5. Membran nach einem der Ansprüche 1 bis 4, enthaltend von 0,1 bis 5 Gew.-% Chitosan, bezogen auf das Gesamtgewicht der Membran.

6. Membran nach einem der Ansprüche 1 bis 5, worin das Chitosan ein gewichtsmittleres Molekulargewicht $M_w$, bestimmt durch GPC-Analyse, im Bereich von 5 bis 375 kDa aufweist.

7. Membran nach einem der Ansprüche 1 bis 6, worin das Chitosan einen Deacetylierungsgrad im Bereich von 85 bis 95% aufweist.

8. Verfahren zur Herstellung einer porösen Hohlfasermembran mit einem darauf immobilisierten Antikoagulans, umfassend die Herstellung einer mikroporösen Hohlfaser-Trägermembran und anschließendes Aufpfropfen eines Antikoagulans auf mindestens eine Oberfläche der Trägermembran, wobei die Herstellung der mikroporösen Hohlfaser-Trägermembran die Schritte umfasst:

   a) Herstellen einer Polymerlösung, die mindestens ein Polysulfon, Polyethersulfon oder Polyarylethersulfon; mindestens ein Polyvinylpyrrolidon; mindestens ein Chitosan; und N-Methyl-2-pyrrolidon enthält;
   b) Extrudieren der Polymerlösung durch einen äußeren Ringschlitz einer Düse mit zwei konzentrischen Öffnungen in ein Fällungsbad; gleichzeitig
   c) Extrudieren einer zentralen Flüssigkeit durch die innere Öffnung der Düse;
   d) Waschen der erhaltenen Membran; anschließend
   e) Trocknen der Membran;

   wobei die Polymerlösung 10 bis 15 Gew.-% Polysulfon, Polyethersulfon oder Polyarylethersulfon enthält, bezogen auf das Gesamtgewicht der Polymerlösung; und 1 bis 10 Gew.-% Polyvinylpyrrolidon, bezogen auf das Gesamtgewicht der Polymerlösung; und 0,05 bis 0,6 Gew.-% Chitosan, bezogen auf das Gesamtgewicht der Lösung.

9. Verfahren nach Anspruch 8, bei dem das Chitosan einen Deacetylierungsgrad von 75% bis 100% und ein gewichtsmittleres Molekulargewicht $M_w$, bestimmt durch GPC-Analyse, im Bereich von 5 kDa bis 375 kDa aufweist.

10. Verfahren nach Anspruch 8 oder 9, bei dem das Herstellen der Polymerlösung das Auflösen des Chitosans in Milchsäure umfasst.

11. Verfahren nach Anspruch 10, bei dem das Chitosan in einer 90 Gew.-%igen wässrigen Milchsäurelösung gelöst wird.

12. Verfahren nach einem der Ansprüche 8 bis 11, bei dem das Antikoagulans auf die mindestens eine Oberfläche der Trägermembran aufgepfropft wird, indem eine wässrige Lösung des Antikoagulans mit der mindestens einen Oberfläche der Trägermembran in Kontakt gebracht wird.

13. Verfahren nach einem der Ansprüche 8 bis 12, wobei das Antikoagulans unfraktioniertes Heparin ist.

**14.** Filtrations- und/oder Diffusionsvorrichtung, die eine Vielzahl von porösen Hohlfasermembranen nach einem der Ansprüche 1 bis 7 umfasst.

**15.** Verwendung einer porösen Hohlfasermembran nach einem der Ansprüche 1 bis 7 in der Hämodialyse, Hämodiafiltration oder Hämofiltration.

**Revendications**

**1.** Membrane à fibres creuses poreuses sur laquelle est immobilisé un anticoagulant ; la membrane comprenant un mélange de i) une polysulfone, une polyéthersulfone ou une polyaryléthersulfone ; ii) une polyvinylpyrrolidone ; et iii) un chitosane.

**2.** Membrane selon la revendication 1, dans laquelle l'anticoagulant est un glycosaminoglycane.

**3.** Membrane selon la revendication 2, dans laquelle l'anticoagulant est de l'héparine non fractionnée.

**4.** Membrane selon l'une quelconque des revendications 1 à 3, dans laquelle la concentration de l'anticoagulant sur la fibre creuse poreuse est comprise entre 1 000 et 5 000 UI/m$^2$.

**5.** Membrane selon l'une quelconque des revendications 1 à 4, comprenant de 0,1 à 5 % en poids, par rapport au poids total de la membrane, de chitosane.

**6.** Membrane selon l'une quelconque des revendications 1 à 5, dans laquelle le chitosane a un poids moléculaire moyen en poids $M_w$, déterminé par analyse GPC, dans la plage de 5 à 375 kDa.

**7.** Membrane selon l'une quelconque des revendications 1 à 6, dans laquelle le chitosane a un degré de désacétylation dans la plage de 85 à 95 %.

**8.** Procédé de préparation d'une membrane à fibres creuses poreuses sur laquelle est immobilisé un anticoagulant, comprenant la production d'une membrane de support à fibres creuses microporeuses et le greffage d'un anticoagulant sur au moins une surface de la membrane de support, la production de la membrane de support à fibres creuses microporeuses comprenant les étapes suivantes :

a) formation d'une solution polymère comprenant au moins une polysulfone, une polyéthersulfone ou une polyaryléthersulfone ; au moins une polyvinylpyrrolidone ; au moins un chitosane ; et de la N-méthyl-2-pyrrolidone ;
b) extruder la solution de polymère à travers une fente annulaire externe d'une buse à deux ouvertures concentriques dans un bain de précipitation ; simultanément
c) extruder un fluide central à travers l'ouverture intérieure de la buse ;
d) laver la membrane obtenue ; ensuite
e) sécher la membrane ;

dans laquelle la solution de polymère comprend de 10 à 15 % en poids, par rapport au poids total de la solution de polymère, de polysulfone, de polyéthersulfone ou de polyaryléthersulfone ; et de 1 à 10 % en poids, par rapport au poids total de la solution de polymère, de polyvinylpyrrolidone ; et de 0,05 à 0,6 % en poids, par rapport au poids total de la solution, de chitosane.

**9.** Procédé selon la revendication 8, dans lequel le chitosane a un degré de désacétylation de 75 % à 100 %, et un poids moléculaire moyen en poids $M_w$, déterminé par analyse GPC, dans la plage de 5 kDa à 375 kDa.

**10.** Procédé selon la revendication 8 ou 9, dans lequel la formation de la solution polymère comprend la dissolution du chitosane dans de l'acide lactique.

**11.** Procédé selon la revendication 10, dans lequel le chitosane est dissous dans une solution aqueuse à 90 % p/p d'acide lactique.

**12.** Procédé selon l'une quelconque des revendications 8 à 11, dans lequel l'anticoagulant est greffé sur la ou les surfaces

**EP 4 225 477 B1**

de la membrane de support par mise en contact d'une solution aqueuse de l'anticoagulant avec la ou les surfaces de la membrane de support.

13. Procédé selon l'une quelconque des revendications 8 à 12, dans lequel l'anticoagulant est de l'héparine non fractionnée.

14. Dispositif de filtration et/ou de diffusion comprenant une pluralité de membranes à fibres creuses poreuses selon l'une quelconque des revendications 1 à 7.

15. Utilisation d'une membrane à fibres creuses poreuses selon l'une quelconque des revendications 1 à 7 en hémodialyse, hémodiafiltration ou hémofiltration.

16

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20030021826 A1
- US 5840190 A
- EP 1024886 A1
- US 10500549 B2
- US 2019076787 A1
- WO 2020144244 A1
- WO 2007069983 A1

### Non-patent literature cited in the description

- **LINHARDT, ROBERT et al.** Immobilization of Heparin: Approaches and Applications. *Current topics in medicinal chemistry*, 01 January 2008, vol. 8 (2), 80-100